# EUROPEAN PATENT SPECIFICATION

(11) **EP 0 806 213 B1**
(45) Date of publication and mention of the grant of the patent: **23.07.2003**
(21) Application number: 97201317.1
(22) Date of filing: 02.05.1997
(51) Int. Cl.: A61M 1/00

(54) **Suction catheter with haemostatic device**
Absaugkatheter mit hämostatischer Vorrichtung
Cathéter à aspiration avec dispositif hémostatique

(30) Priority: 07.05.1996 NL 1003056
(43) Date of publication of application: 12.11.1997
(73) Proprietor: CORDIS EUROPA N.V., NL-9301 LJ Roden (NL)
(72) Inventor: Bosma, Gjalt, 9218 PV Opeinde (NL); Noppert, Tiemen, 9649 EC Muntendam (NL)
(74) Representative: 't Jong, Bastiaan Jacob

(56) References cited:
- EP-A- 0 576 380
- EP-A- 0 654 275
- EP-A- 0 693 295
- WO-A-93/05826
- GB-A- 2 117 497

## Description

The invention relates to a suction catheter as described in the introductory part of claim 1. Such a catheter is known from EP-A-0 693 295.

With such a known suction catheter the jet nozzle may produce a liquid jet along the opening resulting in ejector action. Due to the suction generated at the site of the opening, for instance thrombi may be discharged.

In order to prevent blood from flowing out via the discharge lumen and the pressure lumen when the catheter is positioned inside the body of a patient but is not in use, it is known to shut off the proximal ends of the discharge lumen and the pressure lumen with valves.

When using such a known suction catheter there is a danger that liquid under pressure is introduced into the pressure line whilst the haemostatic valve of the discharge lumen is still closed. In that case the liquid under pressure supplied will enter, via the opening at the distal end, the bloodstream of the patient, which is undesirable. Another type of catheter known, from WO-A-9 305 826, comprises controllable valve means within the flushing and suction channels. These valve means, however, are not capable not are conceived, for addressing or solving the above problem, which is at the basis of the present invention.

The object of the invention is to provide a suction catheter of the type as described in the preamble of claim 1 which does not have this drawback.

This aim is achieved with the suction catheter according to the invention as characterised in claim 1. As the valve means open and close the pressure lumen and the suction lumen simultaneously, the discharge lumen is always open when liquid under pressure is introduced into the pressure line.

A very advantageous further development of the invention has been characterised in claim 2. In this case the valve means shut off the lumens when the catheter is not in use and the valve means are opened automatically when liquid under pressure is supplied.

Preferably the measure as set out in claim 3 is employed. Additionally the activation means act against the force exerted by the resilient means in order to move the valve means into the open position.

A very suitable embodiment has been characterised in claim 4. As soon as liquid under pressure is introduced into the pressure lumen, pressure is applied to this slide due to the pressure of this liquid and is pushed, against the force exerted by the resilient means, into the open position.

The valve means and the operating means can be combined in a favourable manner by employing the measure as set out in claim 5.

A suitable embodiment has additionally been characterised in claim 6. In the open position the discharge lumen forms a continuous channel so that no flow resistance is created, which is very important in order for the catheter to function properly.

With the measure as set out in claim 7 simultaneous opening and closing of the discharge lumen and the pressure lumen is achieved in an advantageous manner.

By employing the measure as set out in claim 8 it is achieved in a simple manner that on the one hand the pressure lumen is closed when the valve means are closed whilst, on the other hand, the liquid under pressure supplied via the relatively proximal section of the pressure lumen can act on the slide in the required manner in order to open it.

A suitable embodiment has been characterised in claim 9.

The invention will be explained in greater detail with reference to the attached drawings.

Figure 1 illustrates in a partly broken away perspective view a preferred embodiment of the catheter according to the invention whilst the valve means are closed.

Figure 2 shows a view corresponding to figure 1 with the valve means open.

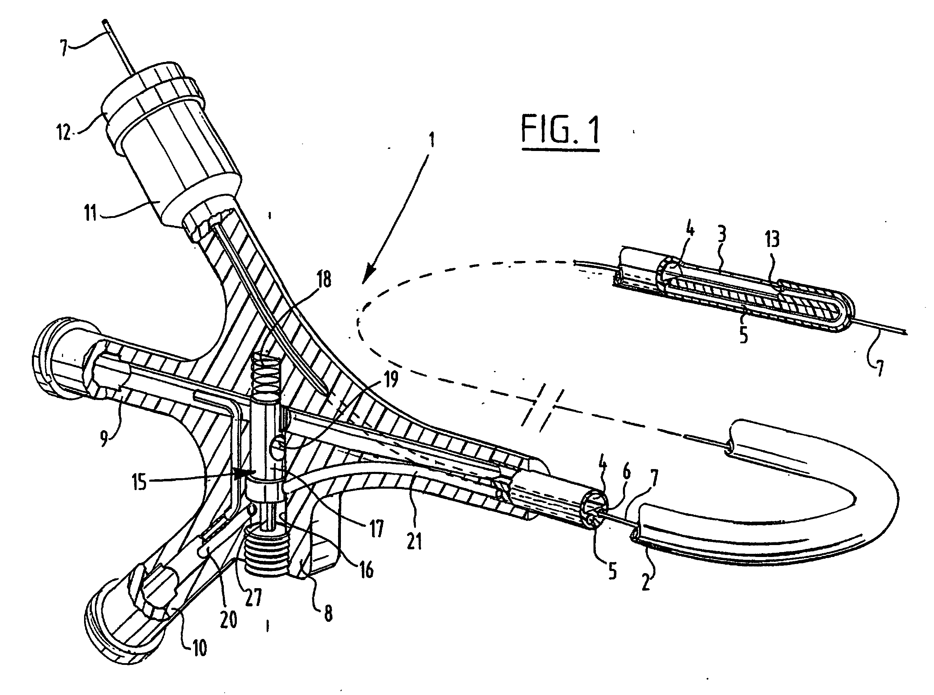

The catheter 1 illustrated in figure 1 comprises a tube-like basic body 2. Three lumens have been formed inside the basic body 2; a pressure lumen 5, a discharge lumen 4 and a guide wire lumen 6.

At the distal end of the basic body 2 an opening 3 has been formed which is connected with the discharge lumen 4. At the distal end of the catheter the pressure lumen 5 ends in a jet nozzle 13. Liquid under pressure supplied via the pressure lumen 5 leaves the jet nozzle 13 in the form of a powerful jet, which is directed inwards along the opening 3 in the discharge lumen 4. Due to this jet there will be an ejector action which generates suction at the opening 3, which can be used to remove for instance blood clots via the discharge lumen 4.

When positioning the catheter 1, the guide wire lumen 6 is used for the passage of the guide wire 7.

At the proximal end of the catheter 1 a connecting member 8 has been arranged, inside of which the connections with the lumens have been received. For the sake of clarity this connecting member 8 has been illustrated in figure 1 whilst rotated, in relation to the basic body 2, a quarter turn to the left. Via a channel 23 in the connecting member 8, the discharge lumen 4 is connected with the discharge connection 9. The pressure lumen 5 is connected with a pressure connection 10 via channel sections 20 and 21. The guide wire channel 7 is connected with the guide wire connection 11. This guide wire connection 11 has been provided with an anti-haemostatic-device 12 known as such, which prevents blood from flowing out via the guide wire channel whilst using the catheter 1.

The discharge connection 9 and the pressure connection 10 have both been provided with Luer-lock connections.

Inside the connecting member 8 valve means 15 have been received which can open and close the pressure lumen and the discharge lumen simultaneously.

With the preferred example of an embodiment illustrated, the valve means 15 comprise a slide 17, movable to and fro, received inside a channel 16 formed inside the connecting member. The channel 16 has been closed off with a plug 22. As can be seen in the figures, the slide 17 is, at its end turned away from the screw plug 22, acted upon by a helical spring 18. The latter pushes the slide 17 into the closed position illustrated in figure 1, in which both the discharge lumen and the pressure lumen have been blocked. The slide has been provided with a distance pin 27 with which the slide 17 is positioned against the screw plug 22 when closed.

As can be seen in figure 1, in closed position the slide 17 leaves the relatively proximal section 20 of the pressure lumen free and closes the relatively distal section 21 of the pressure lumen off. In this state consequently no blood can leak away via the discharge lumen 4 or the pressure lumen 5 from the connections 9 , 10 respectively.

As the relatively proximal section 20 of the pressure lumen is connected to that side of the slide 17 opposite to the side of the slide which is acted upon by the spring 18, the slide 17 will be pushed upwards against the force exerted by the spring 18, when via the pressure connection 10 liquid under pressure is supplied.

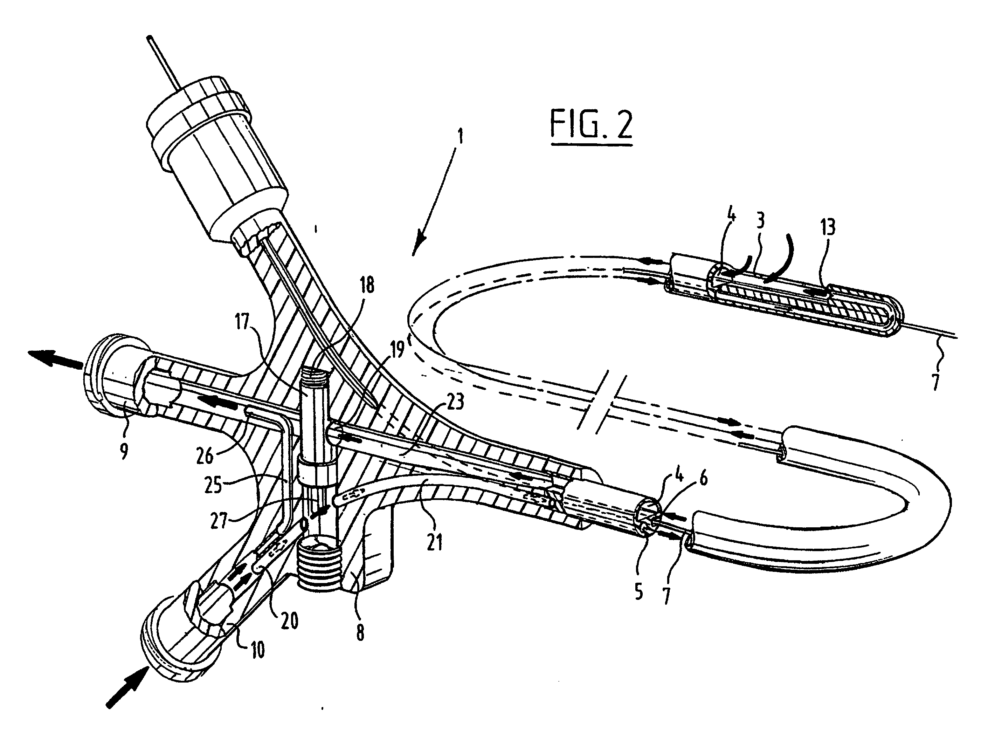

In the open position illustrated in figure 2, a transverse channel 19 in the slide 17 has been moved in line with the channel 23 inside the connecting member 8, forming the continuation of the discharge lumen 4. The cross-section of the transverse channel 19 is equal to the cross-section of the channel 23, so that in the open position there will be no resistance to flow. The material sucked up through the opening 3 can consequently be removed freely via the discharge lumen 4 and the channel section 23 from the discharge connection 9.

At the same time the discharge lumen 4 is opened the slide 17 is moved away from the entrance of the relatively distal section 21 of the pressure lumen, so that the liquid under pressure supplied via the connection 10 can flow freely via the relatively proximal section 20 to the relatively distal section 21 and then via the pressure lumen 5 to the jet nozzle 13.

To assist the flow in the discharge lumen an auxiliary pressure channel 25 has been formed in the connecting member 8 of which the outlet 26 is directed towards the connecting member 9. Via this auxiliary pressure channel 25 part of the liquid under pressure supplied through the pressure connection 10 flows away in the direction indicated by the arrows, as a result of which a liquid jet is formed at the outlet 26 of the auxiliary pressure channel 25, reinforcing the flow in the discharge lumen 4 as referred to above.

As soon as the supply of liquid under pressure via the pressure connection 10 is closed off, the slide 17 will be pushed into the closed position illustrated in figure 1 due to the action of the spring 18, in which at the same time the pressure lumen and the discharge lumen are closed off, so that no blood can leak away via the lumens in an undesirable manner.

## Claims

1. Suction catheter (1) comprising a flexible tube-like basic body (2) with a proximal and a distal end, inside of which at least a pressure lumen (5) and a discharge lumen (4) extend,
a connector (8) at the proximal end respectively connecting the pressure lumen (5) to a pressure connection (10), and the discharge lumen (4) to a discharge connection (9),
an opening (3), positioned in the side wall at the distal end of said catheter (1), in communication with the discharge lumen (4),
a jet nozzle (13) connected with the pressure lumen (5) and opening out into the discharge lumen (4) and arranged to direct pressurized fluid from the pressure lumen (5) inwards along said opening (3) and into said discharge lumen (4) to create a suction at said opening (3), **characterized in that**,
the catheter (1) comprises, at the aproximal end, valve means (15) which can be moved by means of operating means (17) between a closed position in which the pressure lumen (5) and the discharge lumen (4) are closed and an open position wherein both lumens (5, 4) are open.

2. Suction catheter as claimed in claim 1, wherein the operating means comprise pressure detection means (16, 17) connected with the pressure lumen (5) and associated with it activation means which open the valve means (15) when pressure is generated inside the pressure lumen (5).

3. Suction catheter as claimed in claim 2, wherein the valve means (15) are forced into the closed position by resilient means (18).

4. Suction catheter as claimed in claim 3, wherein the operating means comprise a slide (17) received in a movable manner inside a channel (16) which, on one side, is acted upon by the resilient means (18) and is connected with the pressure lumen (5) at the opposite side.

5. Suction catheter as claimed in claim 4, wherein the discharge lumen (4) intersects the channel (16) and the slide (17) in closed position blocks the discharge lumen (4) and leaves it free in open position.

6. Suction catheter as claimed in claim 5, wherein the slide (17) comprises a transverse channel (19) which has substantially the same cross-section as the discharge lumen (4), which in open position is situated in line with the discharge lumen (4).

7. Suction catheter as claimed in one of the claims 4-6, wherein the pressure lumen (5) intersects the channel (16) and the slide (17) blocks the pressure lumen (5) in the closed position and leaves it free in the open position.

8. Suction catheter as claimed in claim 7, wherein, in the closed position, the slide (17) leaves the relatively proximal section of the pressure lumen (5) free and closes off the relatively distal section.

9. Suction catheter as claimed in one of the previous claims, wherein the valve means (15) have been received in a connecting member (8) at the proximal end of the catheter (1).

## Patentansprüche

1. Absaugkatheder (1), umfassend einen flexiblen, röhrenförmigen Grundkörper (2) mit einem proximalen und einem distalen Ende, in dem mindestens ein Drucklumen (5) und ein Abflußlumen (4) sich erstrecken,
einen Verbinder (8) beim proximalen Ende, der jeweils *das* Drucklumen (5) mit einer Druckverbindung (10) und das Abflußlumen (4) mit einer Abflußverbindung (9) verbindet,
eine Öffnung (3), die in der Seitenwand beim distalen Ende des Katheders (1), in kommunzierender Verbindung mit dem Abflußlumen (4), positioniert ist,
eine Strahldüse (13), die mit dem Drucklumen (5) verbunden ist und in das Abflußlumen (4) öffnet und so angeordnet ist, daß unter Druck gesetzte Flüssigkeit vom Drucklumen (5) nach innen entlang der Öffnung (3) und in das Abflußlumen (4) hinein gerichtet ist, um bei der Öffnung (3) ein Absaugen zu bilden, **dadurch gekennzeichnet, daß** der Katheder (1) beim proximalen Ende eine Ventileinrichtung (15) umfaßt, die durch eine Betriebseinrichtung (17) zwischen einer geschlossenen Position, in der das Drucklumen (5) und das Abflußlumen (4) geschlossen sind, und einer geöffneten Position, in der beide Lumen (5, 4) geöffnet sind, bewegt werden kann.

2. Absaugkatheder wie im Anspruch 1 beansprucht, wobei die Betriebseinrichtung eine mit dem Drucklumen (5) verbundene Druckdetektionseinrichtung (16, 17) und, damit verbunden, eine Aktivierungseinrichtung umfaßt, die die Ventileinrichtung (15) öffnet, wenn Druck im Inneren des Drucklumens (5) erzeugt wird.

3. Absaugkatheder wie im Anspruch 2 beansprucht, wobei die Ventileinrichtung (15) in die geschlossene Position durch eine elastische Einrichtung (18) gezwungen wird.

4. Absaugkatheder wie im Anspruch 3 beansprucht, wobei die Betriebseinrichtung ein Gleitelement (17) umfaßt, das auf bewegliche Weise im Inneren eines Kanals (16) aufgenommen ist, auf das auf einer Seite die elastische Einrichtung (18) wirkt und das auf der gegenüberliegenden Seite mit dem Drucklumen (5) verbunden ist.

5. Absaugkatheder wie im Anspruch 4 beansprucht, wobei das Abflußlumen (4) den Kanal (16) schneidet, und wobei das Gleitelement (17) in der geschlossenen Position das Abflußlumen (4) blockiert und es in geöffneter Position frei gibt.

6. Absaugkatheder wie im Anspruch 5 beansprucht, wobei das Gleitelement (17) einen Querkanal (19) umfaßt, der im wesentlichen den gleichen Querschnitt wie das Abflußlumen (4) aufweist, wobei der Querkanal in geöffneter Position sich in einer Linie mit dem Abflußlumen (4) befindet.

7. Absaugkatheder wie in einem der Ansprüche 4 bis 6 beansprucht, wobei das Drucklumen (5) den Kanal (16) schneidet und das Gleitelement (17) das Drucklumen (5) in der geschlossenen Position blockiert und es in der geöffneten Position freigibt.

8. Absaugkatheder wie im Anspruch 7 beansprucht, wobei in der geschlossenen Position das Gleitelement (17) den relativ proximalen Abschnitt des Drucklumens (5) freigibt und den relativ distalen Abschnitt abschließt.

9. Absaugkatheder wie in einem der vorangehenden Ansprüche beansprucht, wobei die Ventileinrichtung (15) in einem Verbindungselement (8) beim proximalen Ende des Katheders (1) eingerichtet ist.

## Revendications

1. Cathéter d'aspiration (1) comprenant un corps de base sous la forme d'un tube flexible (2) avec une extrémité proximale et distale, à l'intérieur duquel s'étendent au moins une lumière de pression (5) et une lumière de décharge (4),
un connecteur (8) au niveau de l'extrémité proximale et raccordant respectivement la lumière de pression (5) à un raccord de pression (10), et la lumière de décharge (4) à un raccord de décharge (9),
une ouverture (3) située dans la paroi latérale à l'extrémité distale dudit cathéter (1), en communication avec la lumière de décharge (4),
un orifice d'éjection (13) raccordé à la lumière de pression (5) et s'ouvrant dans la lumière de décharge (4) et disposé de manière à diriger le fluide sous pression à partir de la lumière de pression (5) vers l'intérieur de ladite ouverture (3) et dans ladite lumière de décharge (4) pour créer une aspiration au niveau de ladite ouverture (3), **caractérisé en ce que**
le cathéter (1) comprend au niveau de l'extrémité proximale une valve (15) qui peut passer au moyen de moyens de commande (17) d'une position fermée dans laquelle la lumière de pression (5) et la lumière de décharge (4) sont fermées, à une position ouverte dans laquelle les deux lumières (5, 4) sont ouvertes.

2. Cathéter d'aspiration selon la revendication 1, dans lequel les moyens de commande comprennent des moyens de détection de la pression (16, 17) raccordés à la lumière de pression (5) et associés avec ses moyens d'activation qui ouvrent la valve (15) lorsqu'une pression est générée à l'intérieur de la lumière de pression (5).

3. Cathéter d'aspiration selon la revendication 2, dans lequel la valve (15) est poussée en position fermée par des moyens résistants (18).

4. Cathéter d'aspiration selon la revendication 3, dans lequel les moyens de commande comprennent un élément coulissant (17) reçu de manière mobile à l'intérieur d'une rainure (16) qui, sur un côté, est actionné par les moyens résistants (18) et est raccordé à la lumière de pression (5) du côté opposé.

5. Cathéter d'aspiration selon la revendication 4, dans lequel la lumière de décharge (4) forme une intersection avec la rainure (16) et l'élément coulissant (17) en position fermée bloque la lumière de décharge (4) et la laisse libre en position ouverte.

6. Cathéter d'aspiration selon la revendication 5, dans lequel l'élément coulissant (17) comprend une rainure transversale (19) qui a sensiblement le même profil en travers que la lumière de décharge (4), qui en position ouverte se trouve alignée avec la lumière de décharge (4).

7. Cathéter d'aspiration selon l'une quelconque des revendications 4 à 6, dans lequel la lumière de pression (5) forme une intersection avec la rainure (16) et l'élément coulissant (17) bloque la lumière de pression (5) en position fermée et la laisse libre en position ouverte.

8. Cathéter d'aspiration selon la revendication 7, dans lequel, en position fermée, l'élément coulissant (17) laisse la partie relativement proximale de la lumière de pression (5) libre et condamne la partie relativement distale.

9. Cathéter d'aspiration selon l'une quelconque des revendications précédentes, dans lequel la valve (15) a été reçue dans un élément de raccordement (8) au niveau de l'extrémité proximale du cathéter (1).
